# EUROPÄISCHE PATENTANMELDUNG

(11) **EP 1 829 975 A1**
(43) Veröffentlichungstag der Anmeldung: **05.09.2007**
(21) Anmeldenummer: 07101893.1
(22) Anmeldetag: 07.02.2007
(51) Int. Cl.: C12P 13/02

(54) **Verfahren zur Herstellung chiralen 2-Hydroxy-4-(methylthio)buttersäureamids durch Nitrilhydratase-katalysierte Hydrolyse des entsprechenden chiralen 2-Hydroxy-4-(methylthio)buttersäurenitrils**

(30) Priorität: 02.03.2006 DE 102006010254
(71) Anmelder: Degussa GmbH, 40474 Düsseldorf (DE)
(72) Erfinder: Effenberger, Franz Prof., 70597 Stuttgart (DE); Bohrer, Anja, 73326 Deggingen (DE); Huthmacher, Klaus Dr., 63571 Gelnhausen (DE); Oßwald, Steffen Dr., 63517 Rodenbach (DE)

(57) **Zusammenfassung**

Die Erfindung betrifft die Umsetzung von D- und/oder L-2-Hydroxy-4-(methylthio)buttersäurenitril zu D- und/oder L-2-Hydroxy-4-(methylthio)buttersäureamid, mit Hilfe der in WO05093080 beschriebenen Nitrilhydratase aus *Rhodococcus opacus* unter Bedingungen, die die Racemisierung der optisch aktiven Reaktanten weitgehend bis vollständig unterbinden.

## Beschreibung

Die Erfindung betrifft die enzymkatalysierte Herstellung von 2-Hydroxy-4-(methylthio)buttersäureamid aus 2-Hydroxy-4-(methylthio)buttersäurenitril.

α-Hydroxycarbonsäuren sind in der Natur weit verbreitet. Sie dienen als Ausgangsverbindungen für die Synthese biologisch aktiver Wirkstoffe im Pharma- und Pflanzenschutzsektor. Von besonderer Bedeutung ist außerdem die enge Beziehung, die zwischen α-Hydroxy- und α-Aminosäuren in biologischen Systemen besteht. Die in biologischen Kreisläufen leicht erfolgenden gegenseitigen Transformationen von α-Hydroxy- in α-Aminosäuren lassen den Schluss zu, dass beide Verbindungsklassen bei manchen Anwendungen zu vergleichbaren Ergebnissen führen. Besonderes Interesse kommt in diesem Zusammenhang denjenigen α-Hydroxycarbonsäuren zu, die den proteinogenen α-Aminosäuren entsprechen. So können Hydroxyanaloga einiger essentieller α-Aminosäuren der Nahrung zugesetzt werden und als Ersatz für die jeweilige α-Aminosäure dienen. Die Synthese von α-Hydroxycarbonsäuren stellt somit für industrielle Anwender eine interessante Alternative zur Synthese der korrespondierenden α-Aminosäuren dar. Insbesondere von Interesse sind in diesem Zusammenhang proteinogene Aminosäuren, die als Futtermitteladditiv Verwendung finden, wie z.B. Methionin oder das Methioninhydroxyanaloge (MHA = 2-Hydroxy-4-(methylthio)buttersäure, HMB).

Ein wichtiger Punkt bezüglich der biologischen Aktivität von α-Aminosäuren ist die Stereochemie. In Proteinen kommen α-Aminosäuren praktisch ausschließlich in der L(S)-Konfiguration vor.

Es ist daher von Interesse, stereoselektive Verfahren zur präparativen Darstellung von α-Hydroxycarbonsäuren zu entwickeln, insbesondere zur Darstellung solcher α-Hydroxycarbonsäuren, die strukturell den proteinogenen α-Aminosäuren entsprechen. Wichtige Zwischenprodukte bei der Synthese der α-Hydroxycarbonsäuren sind die korrespondierenden α-Hydroxycarbonsäureamide, wie z.B. D,L-2-Hydroxy-4-(methylthio)buttersäureamid (D,L-HMB-amid) auf dem Weg zu D,L-2-Hydroxy-4-(methylthio)buttersäure.

Diese werden bisher durch Hydrolyse der entsprechenden α-Hydroxycarbonsäurenitrile nach einer der folgenden prinzipiellen Vorgehensweisen hergestellt:
a) Hydrolyse unter basischen Bedingungen
b) Schwermetall-katalysierte Hydrolyse
c) Hydrolyse unter sauren Bedingungen
d) Enzymatische Hydrolyse

Die Möglichkeit a), Cyanhydrine unter basischen Bedingungen zu verseifen, ist seit langem bekannt. Eine Methode in diesem Bereich ist die Umsetzung in wässriger ammoniakalischer Wasserstoffperoxidlösung (Stamicarbon, B.V.; Jpn. Kokai, Tokkyo Koho JP 62, 178, 555; Chem. Abstr. 1988, 109, 92286). Auch Alkali- und Erdalkaliborate werden für die Hydrolyse von Cyanhydrinen verwendet (AT358552). Allerdings bietet dieser Weg allgemein nur Zugang zu racemischen α-Hydroxycarbonsäureamiden, da die optisch aktiven Cyanhydrine, die als Ausgangsverbindungen dienen sollen, unter basischen Bedingungen sehr schnell racemisieren.

Bei der Schwermetall katalysierten Hydrolyse von Cyanhydrinen (b) wird Mangandioxid am häufigsten als Katalysator eingesetzt, dessen Eigenschaften durch verschiedene Modifikationen variiert werden können. Allerdings liegen auch hier keine Angaben zur Darstellung optisch aktiver α-Hydroxycarbonsäureamide vor, es werden lediglich racemische Produkte beschrieben. Nachteilig ist auch der Einsatz von Schwermetall, da selbst minimale Katalysatorreste in den Endprodukten unerwünscht sind und eine Reinigung entsprechend aufwendig ist.

Die säurekatalysierte Verseifung (c) von optisch aktiven α-Hydroxycarbonsäurenitrilen stellt bislang die am häufigsten verwendete Methode zu Darstellung optisch aktiver α-Hydroxycarbonsäureamide dar. Diese Möglichkeit wurde durch die einfache Zugänglichkeit optisch aktiver Cyanhydrine über die Hydroxynitril Lyase (HNL) katalysierte Addition von Blausäure an Aldehyde eröffnet. Für die säurekatalysierte Verseifung sind verschiedene Reaktionsbedingungen unter Verwendung von Salzsäure, Schwefelsäure oder Essigsäure bekannt. Dabei hat sich als Reaktionsmedium eine auf 0 °C gekühlte 1:1 Mischung aus konz. Salzsäure und Essigsäure bewährt (Rochowski, A., Dissertation, Univ. Stuttgart 1997). Der Nachteil bei diesen Umsetzungen liegt zum einen darin, dass oft ein erheblicher Anteil des Amids zur Säure weiterreagiert und so Produktgemische entstehen und zum anderen darin, dass säureempfindliche Verbindungen nicht umgesetzt werden können. Auch die entstehenden Salze können vor allem bei Anwendungen im technischen Maßstab zu Problemen führen. Ein weiterer Nachteil für die industrielle Anwendung ist die Notwendigkeit der Verwendung teurer säureresistenter Werkstoffe.

Es wurden deshalb insbesondere von industriellen Anwendern vielfältige Untersuchungen vorgenommen, die Nitrilverseifung enzymatisch mit Nitrilasen bzw. Nitrilhydratasen (NHasen) durchzuführen (Möglichkeit d)). Das pH-Optimum von industriell verwendbaren Nitrilasen bzw. Nitrilhydratasen liegt in der Regel deutlich im alkalischen Bereich (pH 8-9), in dem es jedoch zu einer raschen Racemisierung optisch aktiver Cyanhydrine kommt. Es ist daher bisher für wenig sinnvoll erachtet worden, optisch aktive Cyanhydrine für die enzymatische Verseifung einzusetzen. Stattdessen geht man eher von racemischen Cyanhydrinen aus und macht sich die Stereoselektivität, die den meisten Enzymen aufgrund der räumlichen Struktur ihrer Bindungstasche eigen ist, zunutze. Die stereoselektiven Nitrilasen bzw. Nitrilhydratasen setzen aus der racemischen Mischung nur ein Enantiomer um, und es kommt zu einer kinetischen Racematspaltung in Richtung der optisch aktiven α-Hydroxycarbonsäuren bzw. α-Hydroxycarbonsäureamide. Dieser Weg wurde von einer japanischen Arbeitsgruppe erfolgreich durchgeführt (Yamamoto et al., Appl Env Microbiol 1991, Vol. 57, No. 10, p. 3028-3032).

Die kinetischen Racematspaltungen durch Nitrilasen bzw. Nitrilhydratasen haben jedoch den Nachteil, dass sie in der Regel vergleichsweise niedrige Raum/Zeit-Ausbeuten liefern, was vor allem für die Anwendung auf technische Prozesse ein limitierender Faktor ist.

Wesentlich höhere Raum/Zeit-Ausbeuten können hingegen mit Nitrilhydratasen erreicht werden, die nicht stereospezifisch wirken, wie erste Ergebnisse bezüglich der enzymatischen Verseifung ausgehend von optisch aktiven Cyanhydrinen belegen (WO04076385). Die Hydrolyse erfolgte hier mit Wildtypstämmen von *Rhodococcus erythropolis* oder mit den in *E. coli* klonierten rekombinanten Enzymen. Hierbei lag allerdings der Schwerpunkt auf der Darstellung von optisch aktiven α-Hydroxycarbonsäuren mittels eines Nitrilhydratase/Amidase-Systems, wobei α-Hydroxycarbonsäureamide als Zwischenprodukte auftraten, die zu den entsprechenden α-Hydroxycarbonsäuren weiter umgesetzt wurden. Als Substrate dienten vor allem aromatische Substrate wie Chlormandelonitril. Die Hydrolyse von L-HMB-nitril mit einem solchen System wurde nicht durchgeführt.

In US2004/0209345 wird eine nicht stereoselektive Nitrilhydratase aus *Rhodococcus sp.* Cr4 beschrieben, mit der Cyanhydrine, insbesondere 2-Hydroxy-4-(methylthio)-buttersäurenitril (HMB-nitril), in die korrespondierenden Amide überführt werden können. Da hier keine optisch aktiven Substrate und keine stereoselektiven Enzyme eingesetzt werden, liegt das produzierte HMB-Amid in diesem Fall nicht in optisch aktiver Form vor. Die Umsetzung findet dort bevorzugt bei 45 °C und unter Zusatz von 20 %(v/v) Methanol sowie 1-2 mM Ni- oder CoCl₂ statt. In der besagten Anmeldung werden zwar keine Ausbeuten angegeben, es lässt sich aber berechnen, dass im Falle des HMB-nitrils nach 15 min bei 20 °C weniger als 20 % des Substrats zum Amid umgesetzt werden (Beispiele: 1.,10.). Um einen schnelleren Umsatz und eine höhere Ausbeute zu erzielen, muss bei deutlich höheren Temperaturen, z.B. bei 45 °C, gearbeitet werden (Beispiele: 10.). Dies führt zu einem erhöhten Energieverbrauch, was neben der niedrigen Ausbeute und dem hohen Durchsatz an Lösungsmittel und Schwermetallsalzen für die industrielle Herstellung von HMB-Amid von Nachteil ist.

In WO05093080 wird eine nicht stereoselektive Nitrilhydratase aus einem weiteren Rhodococcus-Stamm, Rhodococcus *opacus,* beschrieben, mit deren Hilfe racemische α-Aminonitrile zu α-Aminoamiden umgesetzt werden können. Die beschriebene Nitrilhydratase liefert bei der Hydrolyse von α-Aminonitrilen wesentlich bessere Ausbeuten und es wird unter den dort angegebenen Bedingungen auch eine Hydrolyseaktivität gegenüber dem Nitril des Methioninhydroxyanalogs (HMB-Amid) angegeben. In einem Beispiel für die Hydrolyse von D,L-2-Amino-4-(methylthio)buttersäurenitril (D,L-AMB-nitril) wird zwar ein Umsatz angegeben, aber eine konkrete Ausbeute für das D,L-2-Amino-4-(methylthio)buttersäureamid (D,L-AMB-amid) geht daraus nicht hervor. Die Umsetzung wird bei einem pH von 7,5 durchgeführt.

Für die Umsetzung von α-Hydroxynitrilen werden jedoch keine Bedingungen offenbart. Es wird lediglich eine spezifische Aktivität der Nitrilhydratase für HMB-nitril von nur ca.40 % im Vergleich zu AMB-nitril angegeben. Die für den Umsatz von HMB-nitril zu erwartende Ausbeute ist deshalb deutlich geringer als für die Umsetzung von AMB-nitril. WO05093080 gibt für die Umsetzung von α-Aminonitrilen einen bevorzugten pH-Bereich von 6,8 bis 7,9 an. Aus eigenen Untersuchungen ist bekannt, dass eine Umsetzung von optisch aktivem HMB-nitril analog zu den in WO05093080 genannten Bedingungen zu niedrigen optischen Ausbeuten also merklicher Racemisierung führt. Bei der Anwendung des in WO05093080 beschriebenen Verfahrens auf die Hydrolyse von HMB-nitril ergeben sich also zwei gravierende Mängel: zum einen die niedrige Ausbeute und zum anderen die Racemisierung.

Die Aufgabe, die sich die Erfinder vor dem Hintergrund der Nachteile des Standes der Technik gestellt hatten, war es, ein Verfahren für die Synthese von optisch aktivem HMB-amid aufzufinden, das bei niedrigeren Temperaturen, bzw. niedrigerem Energieverbrauch höhere Ausbeuten liefert und geringere Umsatzzeiten benötigt, als die aus dem Stand der Technik bekannten Verfahren und das im Gegensatz zum Stand der Technik optisch aktives HMB-amid ohne Racemisierung liefert.

Die Aufgabe wurde erfindungsgemäß gelöst, indem ein Verfahren zur enzymatischen Herstellung von D- und/oder L-2-Hydroxy-4-(methylthio)buttersäureamid aus D- und/oder L-2-Hydroxy-4-(methylthio)buttersäurenitril gefunden wurde, das folgende Schritte enthält:
a) Umsetzung des Nitrils mit einem Enzym aus *Rhodococcus opacus,* das Nitrilhydratase-Aktivität aufweist und Untereinheiten mit Aminosäuresequenzen enthält, die zu mindestens 90 % mit den Sequenzen SEQ ID No:1 und SEQ ID NO:2 identisch sind, in wasserhaltigem Medium bei pH-Werten von 6,0 bis 6,7, und
b) gegebenenfalls Abtrennung des Amids.

Bei D- und/oder L-2-Hydroxy-4-(methylthio)buttersäurenitril bzw. -amid handelt es sich bevorzugt um Gemische, die überwiegend, d.h. zu mehr als 50 % bis zu maximal 100 %, das D- oder L-Enantiomer enthalten. Es ist aber auch möglich, das racemische Gemisch von D,L-2-Hydroxy-4-(methylthio)buttersäurenitril zum entsprechenden Gemisch von D,L-2-Hydroxy-4-(methylthio)buttersäureamid umzusetzen.

Das genannte, eine Nitrilhydratase-Aktivität aufweisende Enzym ist mit dem in WO05093080 beanspruchten Enzym identisch, d.h. dass es Untereinheiten enthält, deren Aminosäuresequenzen zu mindestens 90 % und besonders bevorzugt zu mindestens 91 %, 95 %, 97 % oder 99 % mit den Sequenzen SEQ ID No:1 und SEQ ID N0:2 (entsprechen SEQ ID No:2 und SEQ ID No:3 aus WO5093080) identisch sind.

Überraschenderweise wurde festgestellt, dass ein Verfahren mit den oben genannten Schritten, welches bei pH 6,0 bis 6,7 durchgeführt wird, in hoher Ausbeute und gleichzeitig kurzer Reaktionszeit die racemisierungsfreie Umsetzung von D- und/oder L-HMB-nitril zum entsprechenden D- und/oder L-HMB-amid ermöglicht. Das heißt, bei Umsetzung von L-2-Hydroxy-4-(methylthio)buttersäurenitril wurde nach der Reaktion praktisch nur L-2-Hydroxy-4-(methylthio)buttersäureamid gefunden.

Dies ist als wesentliche Verbesserung gegenüber dem Stand der Technik zu sehen, weil bisher kein Verfahren bekannt war, das mit hohen Ausbeuten, in kurzen Reaktionszeiten und bei niedrigen Temperaturen, vorzugsweise Raumtemperatur, racemisierungsfrei zum D- und/oder L-2-Hydroxy-4-(methylthio)buttersäureamid geführt hätte. So wird es beispielsweise durch das erfindungsgemäße Verfahren ermöglicht, bei Reaktionszeiten von nur 15 min Ausbeuten von ≥ 87 % vorzugsweise ≥ 89 % an HMB-amid zu erhalten. Durch unwesentliche Verlängerung der Reaktionszeit lässt sich die Ausbeute auch noch weiter steigern, z.B. auf ≥ 90 % vorzugsweise ≥ 95 %. Je nach Enantionmerenreinheit des Edukts (z.B. 96 % ee D-HMB-nitril) wird eine entsprechende Enantiomerenreinheit im Produkt wiedergefunden (z.B. 96 % ee D-HMB-amid).

Bevorzugt ist für die erfindungsgemäße Verseifung von D-und/oder L-2-Hydroxy-4-(methylthio)buttersäurenitril zu D-und/oder L-2-Hydroxy-4-(methylthio)buttersäureamid der pH-Bereich von 6,2 bis 6,6, besonders bevorzugt von 6,4 bis 6,5 zu verwenden.

Nach der Umsetzung kann das gebildete Amid aus der Reaktionslösung wie bekannt abgetrennt und aufgereinigt werden.

Die erfindungsgemäße Umsetzung kann bei Temperaturen von 5 bis 40 °C durchgeführt werden. In der Regel wird die erfindungsgemäße Reaktion jedoch bei Temperaturen von 10 bis 35 °C, vorzugsweise von 15 bis 30 °C und besonders bevorzugt bei 20 bis 25 °C durchgeführt.

Zur Verbesserung der Löslichkeit der Reaktanten können ein oder mehrere Lösungsmittel als Cosolvens zugesetzt werden, die wenigstens teilweise mit Wasser mischbar sind. Dabei muß das jeweilige Cosolvens zum einen das Cyanhydrin ausreichend lösen und zum anderen die Enzymaktivität so wenig wie möglich beeinträchtigen.

Solche mit Wasser mischbare Lösungsmittel sind polare Lösungsmittel, wobei sowohl polare aprotische als auch polare protische Lösungsmittel zum Einsatz kommen können.

Polare aprotische Lösungsmittel, die sich für die Erhöhung der Löslichkeit von HMB gut eignen sind Dimethylsulfoxid (DMSO), Dimethylformamid (DMF) und Diisopropylether (DIPE).

Als polare protische Lösungsmittel eignen sich bevorzugt C₁-C₄-Alkylalkohole, besonders bevorzugt Methanol, Ethanol, Propanol und iso-Propanol.

Zur erfolgreichen Durchführung des erfindungsgemäßen Verfahrens sollte der Lösungsmittelanteil bezogen auf das Gesamtvolumen der Reaktionslösung bei Verwendung von wenigstens teilweise mit Wasser mischbaren Lösungsmitteln weniger als 20 % (v/v), vorzugsweise maximal 10 %(v/v), insbesondere maximal 5 %(v/v) betragen.

Alternativ zu den mit Wasser mischbaren Lösungsmitteln können auch Lösungsmittel zum Einsatz kommen, die nicht oder nur sehr schlecht mit Wasser mischbar sind. In diesem Fall wird die Reaktion in einem Zweiphasensystem Wasser/organische Lösungsmittel durchgeführt.

Neben den erwähnten Lösungsmitteln können auch ganz andere Lösungsvermittler wie beispielsweise Tenside, Phasentransferkatalysatoren, etc. zum Einsatz kommen.

Die enzymatische Hydrolyse erfolgt erfindungsgemäß bevorzugt in Anwesenheit eines gemäß WO05093080, insbesondere der Beispiele 3,5 und 6, transformierten *E. coli* Stammes.

Die Verwendung eines heterologen Expressionssystems hat den Vorteil, dass aufgrund einer höheren Zielproteinmenge pro Zelle eine höhere spezifische Aktivität pro mg Zelltrockengewicht erreicht werden kann. Es muss aber nicht zwingend mit einem rekombinanten System gearbeitet werden. Eine Umsetzung in Anwesenheit von das erfindungsgemäße Enzym enthaltendem *Rhodococcus opacus* ist ebenso möglich.

Unter "rekombinant" wird in der Fachwelt ein System verstanden in dem Gensequenzen aus verschiedenen Organismen neu miteinander kombiniert werden.

Als Expressionswirt kann nicht nur *E. coli* dienen, sondern jeder Mikroorganismus für den Promotorsequenzen bekannt und zugänglich sind und der Nukleotidsequenzen, z.B. Expressionsplasmide, auf irgendeine Weise aufnehmen kann.

Gängige Wirte für die heterologe Expression sind zum Beispiel verschiedene Hefen, wie *Saccharomyces, Aspergillus* oder Pichia und Bakterien, wie *Bacillus, Caulobacter, Pseudomonas* oder *Streptomyces.* Besonders häufig eingesetzt werden *Corynebacteriaceae* und *Enterobacteriaceae,* bei letzteren insbesondere *Escherichia coli.*

Mit dem Ausdruck "transformierte Zellen" sind im Folgenden Zellen gemeint, die fremde Nukleotidsequenzen, beispielsweise Expressionsplasmide, aufgenommen haben und aufgrund dieser zusätzlichen genetischen Information die Fähigkeit besitzen, katalytisch aktive Nitrilhydratase zu synthetisieren, wobei die Nitrilhydratase Aminosäuresequenzen enthält, die zu mindestens 90 % und besonders bevorzugt zu mindestens 91 %, 95 %, 97 % oder 99 % mit den Sequenzen SEQ ID No:1 und SEQ ID NO:2 identisch sind.

Gängige Methoden für die Herstellung von transformierten Zellen findet der Fachmann in Standardwerken, wie z.B. dem Handbuch von Sambrook, Russel et al.: Molecular Cloning, A Laboratory Manual (Cold Spring Harbor Laboratory Press, 2005) .

Zellen oder transformierte Zellen, die das genannte Protein exprimieren, können in praktisch jeder denkbaren Form für die Katalyse eingesetzt werden, zum Beispiel als lyophilisierte oder ruhende Zellen (sog. *resting cells)* oder auch als gemahlene oder anderweitig aufgeschlossene Zellen. Es ist ebenfalls möglich die Zellen mit gängigen Methoden, beispielsweise mit Hilfe der Kalzium-Alginat-Methode, die u.a. in US005866379A Beispiel 5 beschrieben wird, zu immobilisieren und diese immobilisierten Zellen für die Katalyse einzusetzen.

Weiterhin ist es möglich, die Nitrilhydratase sowohl aus *Rhodococcus opacus* als auch aus einem heterologen Expressionsstamm, wie dem in WO05093080 beschriebenen, mit gängigen Methoden zu isolieren und das isolierte oder teilweise isolierte Enzym für die Katalyse einzusetzen. "Isolieren" bedeutet in diesem Zusammenhang aus der zellulären Umgebung abtrennen. "Teilweise isoliert" ist das Enzym, wenn es angereichert vorliegt, d.h. einen größeren Gewichtsanteil des Gesamtproteins ausmacht, als direkt nach der Zellernte.

Das isolierte oder teilweise isolierte Enzym kann in freier oder immobilisierter Form für die Katalyse eingesetzt werden, wobei die Immobilisierung mit Hilfe bekannter Methoden erreicht werden kann.

Das als Substrat eingesetzte, optisch aktive HMB-nitril kann nach bekannten Methoden, z.B. durch Hydroxynitril Lyase (HNL) katalysierte Addition von Blausäure an den Aldehyd 3-Methylthiopropanal, hergestellt werden.

Für die Ausführung des erfindungsgemäßen Verfahrens kann prinzipiell folgendermaßen vorgegangen werden:
Es wird ein Enzym in rekombinanter Form eingesetzt. Dafür werden die entsprechenden Gene z.B. in E. coli Wirtszellen eingebracht und überexprimiert. Dabei kann eine deutlich höhere Aktivität als bei den Wildtypstämmen erzielt und die unerwünschte Aktivität von Amidasen ausgeschaltet werden.

Für die Umsetzungen werden die aus der Fermentation erhaltenen Zellen in wässrigen Pufferlösungen, bevorzugt Kaliumphosphatpuffer suspendiert. Dabei sollte der pH-Wert in einem Bereich von bei 6,0 bis 6,7, vorzugsweise bei 6,2 bis 6,6 und besonders bevorzugt bei 6,4- 6,5 liegen.

Anschließend wird die so erhaltene Suspension mit dem HMB-nitril versetzt und ggf. das Cosolvens zugegeben.

Die enzymatische Hydrolyse wird bei einer Temperatur von 5 bis 40 °C, bevorzugt vorzugsweise bei 10 bis 35°C, insbesondere bei 15 bis 30 °C, besonders bevorzugt bei 20 bis 25 °C durchgeführt.

Nach erfolgter Hydrolyse zum optisch aktiven HMB-amid erfolgt dessen Isolierung durch Abzentrifugation der Zellen sowie Extraktion der überstehenden wässrigen Lösung mit beispielsweise Essigsäureethylester. Gegebenenfalls erfolgt weitere Reinigung durch Umkristallisation.

Durch die Verwendung von transformierten E. coli wird das optisch aktive Cyanhydrin HMB-nitril auf einfache und effiziente Weise unter milden Bedingungen in das korrespondierende optisch aktive HMB-amid überführt, wobei praktisch keine Racemisierung auftritt.

So gewonnenes optisch aktives HMB-amid kann jederzeit durch bekannte saure oder auch enzymatische Hydrolysemethoden zur entsprechenden optisch aktiven 2-Hydroxy-4-methylthiobuttersäure (HMB) weiterumgesetzt werden. So wird beispielsweise durch Hydrolyse von auf dem erfindungsgemäßen Weg erhaltenem L-2-Hydroxy-4-methylthiobuttersäureamid die entsprechende L-2-Hydroxy-4-methylthiobuttersäure gewonnen, die direkt als Futtermitteladditiv verwendet werden kann. Ein Hydrolyseverfahren unter Verwendung von wässriger Schwefelsäure ist beispielsweise in EP0770061 beschrieben.

Die folgenden Beispiele dienen der näheren Erläuterung der Erfindung, sollen jedoch in keiner Weise beschränkend wirken.

### Beispiel 1

### a) Herstellung der Zellsuspension:

Die aus der Fermentation gemäß WO05093080, insbesondere S. 12 Absatz [0086], erhaltenen Zellen wurden geerntet und in 50 mM Kaliumphosphatpuffer pH 6,5 resuspendiert. Dabei wurde das Puffervolumen so gewählt, dass die optische Dichte der Zellsuspension bei 600 nm zwischen 30 und 60 lag.

### b) Enzymreaktion

232,4 mg R-2-Hydroxy-4-methylthiobuttersäurenitril (D-HMB-nitril) (96 % ee) wurden in 250 µl Methanol gelöst und zu einem 5 ml Aliquot der Zellsuspension gegeben. Anschließend wurde die Reaktionslösung 15 min lang bei Raumtemperatur gerührt. Nach beendeter Reaktion wurden die Zellen abzentrifugiert (10 min, 14000 g) und der Überstand wurde dreimal mit 50 ml Essigester extrahiert. Die vereinigten organischen Phasen wurden über MgS0₄ getrocknet und das Lösungsmittel wurde im Vakuum entfernt.

### c) Bestimmung von Isomerenverhältnissen

10 mg des Rohproduktes aus der Enzymreaktion wurden in 500 µl Methyltertiärbutylether (MTBE) gelöst, mit 100 µl Acetanhydrid und 100 µl Pyridin versetzt und 8 h bei 60 °C derivatisiert. Anschließend wurde die Lösung über eine Kieselgelsäule filtriert (3 x 0,5 cm; ca. 4 ml Essigester zur Elution). Das Eluat wurde direkt zur gaschromatographischen Bestimmung des Umsatzes und der Enantiomerenverhältnisse der acetylierten α-Hydroxycarbonsäureamide verwendet. Als Vergleich dienten die jeweiligen acetylierten Racemate.

### d) Gaschromatographische Reaktionsverfolgung

In Zeitintervallen von einigen Minuten wurden der Reaktionslösung (siehe Beispiel 1 b)) 200 µl Proben entnommen. Diese wurden mit je 2 ml MTBE versetzt und über MgSO₄ getrocknet, um überschüssiges Wasser zu binden. Die Etherphase wurde abdekantiert und wie in Beispiel 1 c) beschrieben derivatisiert. Gaschromatographisch wurden nun Umsatz und Enantiomerenverhältnis bestimmt. Der Zeitpunkt des Abbruchs der Reaktion wurde dann festgelegt, wenn kein weiterer Umsatz feststellbar war.

Nach 15 min war die Reaktion nahezu vollständig abgelaufen. Es wurden 259,4 mg/5 ml R-2-Hydroxy-4-methylthiobuttersäureamid gebildet, was einer Ausbeute von 87 % entspricht. Die Enantiomerenreinheit des HMB-amids lag mit 96 % genauso hoch wie die des Substrates (HMB-nitril, 96 % ee), d.h. die Umsetzung erfolgte praktisch racemisierungsfrei.

### Beispiel 2

232,4 mg S-2-Hydroxy-4-methylthiobuttersäurenitril (L-HMB-nitril) (89 % ee) wurden analog zu Beispiel 1 umgesetzt. In einer Reaktionszeit von 15 min wurden 89 % des L-HMB-nitrils zu L-HMB-amid (89 % ee) umgesetzt, d.h. die Reaktion erfolgte auch hier praktisch racemisierungsfrei.

## Patentansprüche

1. Verfahren zur enzymkatalysierten Herstellung von D-und/oder L-2-Hydroxy-4-(methylthio)buttersäureamid aus D- und/oder L-2-Hydroxy-4-(methylthio)buttersäurenitril, das folgende Schritte enthält:
a) Umsetzung des Nitrils mit einem Enzym aus *Rhodococcus opacus,* das Nitrilhydratase-Aktivität aufweist und Untereinheiten mit Aminosäuresequenzen enthält, die zu mindestens 90 % mit den Sequenzen SEQ ID No:1 und SEQ ID NO:2 identisch sind, in wasserhaltigem Medium bei pH-Werten von 6,0 bis 6,7, und
b) gegebenenfalls Abtrennung des Amids.

2. Verfahren gemäß Anspruch 1, **dadurch gekennzeichnet, dass** die Umsetzung in wasserhaltigem Medium bei pH-Werten von 6,2 bis 6,6, insbesondere 6,4 bis 6,5 durchgeführt wird.

3. Verfahren gemäß Anspruch 1 oder 2, **dadurch gekennzeichnet, dass** die Umsetzung bei Temperaturen von 5 bis 40 °C durchgeführt wird.

4. Verfahren gemäß Anspruch 3, **dadurch gekennzeichnet, dass** die Umsetzung bei Temperaturen von 10 bis 35 °C, vorzugsweise 15 bis 30 °C und insbesondere 20 bis 25 °C durchgeführt wird.

5. Verfahren gemäß einem der vorhergehenden Ansprüche, **dadurch gekennzeichnet, dass** die Umsetzung in Gegenwart eines oder mehrerer wenigstens teilweise mit Wasser mischbaren Lösungsmittels durchgeführt wird.

6. Verfahren gemäß Anspruch 5, **dadurch gekennzeichnet, dass** als Lösungsmittel polare aprotische Lösungsmittel oder polare protische Lösungsmittel eingesetzt werden.

7. Verfahren gemäß Anspruch 6, **dadurch gekennzeichnet, dass** als polare aprotische Lösungsmittel Dimethylsulfoxid (DMSO), Dimethylformamid (DMF) oder Diisopropylether (DIPE) eingesetzt werden.

8. Verfahren gemäß Anspruch 6, **dadurch gekennzeichnet, dass** als polare protische Lösungsmittel C₁-C₄-Alkylalkohole eingesetzt werden.

9. Verfahren gemäß Anspruch 8, **dadurch gekennzeichnet, dass** Methanol, Ethanol, Propanol oder iso-Propanol als polare protische Lösungsmittel verwendet werden.

10. Verfahren gemäß einem der Ansprüche 5 bis 9, **dadurch gekennzeichnet, dass** weniger als 20 %(v/v), vorzugsweise maximal 10 %(v/v), insbesondere maximal 5 %(v/v) Lösungsmittel bezogen auf das Gesamtvolumen der Reaktionslösung eingesetzt werden.

11. Verfahren gemäß einem der vorhergehenden Ansprüche, **dadurch gekennzeichnet, dass** das Enzym gemäß Anspruch 1 in isolierter oder in teilweise isolierter Form, in freier oder immobilisierter Form als Katalysator eingesetzt wird.

12. Verfahren gemäß Anspruch 1 bis 10, **dadurch gekennzeichnet, dass** Zellen oder transformierte Zellen, die das Enzym gemäß Anspruch 1 exprimieren, in ruhender, lyophilisierter, immobilisierter oder aufgeschlossener Form als Katalysator eingesetzt werden.

13. Verfahren gemäß Anspruch 12, **dadurch gekennzeichnet, dass** transformierte Zellen aus einem Mikroorganismenstamm ausgewählt aus der Gruppe umfassend die Hefen *Saccharomyces, Aspergillus* und *Pichia* und die Bakterien *Bacillus, Caulobacter, Pseudomonas, Streptomyces, Corynebacteriaceae* und *Enterobacteriaceae* eingesetzt werden.

14. Verfahren gemäß Anspruch 13, **dadurch gekennzeichnet**, das transformierte *Enterobacteriaceae* der Spezies *E. coli* eingesetzt werden.


SEQUENCE LISTING
<110>  Degussa AG

<120>  Enzymkatalysierte Hydrolyse von optisch aktivem 2-Hydroxy-
4-(methylthio)buttersäurenitril

<160>  2
<300>
<310>  DE 10 2004 013 824
<311>  20.03.2004
<312>  13.10.2005

<210>  1
<211>  235
<212>  PRT
<213>  Rhodococcus opacus

<400>  1

Met Asn Gly Ile Phe Asp Leu Gly Gly Thr Asp Gly Met Gly Pro Val
1               5                   10                  15

Asp Asn Asp Lys Gly Thr Glu Pro Val Phe Arg Ser Ala Trp Glu Lys
            20                  25                  30

Ala Ala Phe Ser Met Phe Ala Gln Gly Ala Arg Ala Gly Leu Tyr Asn
        35                  40                  45

Ile Asp Glu Phe Arg His Cys Val Glu Gln Met Asp Pro Ala Glu Tyr
    50                  55                  60

Leu Leu Ser Asn Tyr Tyr Glu His Trp Thr His Ala Val Glu His Phe
65                  70                  75                  80

Ala Gln Gln Lys Asn Leu Ile Thr Ala Ala Glu Leu Glu Lys Arg Thr
                85                  90                  95

His Phe Tyr Arg Asp Asn Pro Glu Ala Pro Leu Pro Glu Arg Lys Asp
            100                 105                 110

Pro Glu Leu Leu Asp Phe Val Asn Thr Ala Ile Ala Asn Gly Phe Ala
        115                 120                 125

Ala Ser Arg Glu Thr Asn Arg Ser Ala Ala Phe Thr Ile Gly Asp Gln
    130                 135                 140

Val Leu Ile Ala Ala Asp Ser Pro Phe Gly His Thr Arg Arg Ala Gly
145                 150                 155                 160

Tyr Ile Arg Gly Lys Thr Gly Val Ile Thr Ala Thr His Gly Ala Tyr
                165                 170                 175

Val Tyr Pro Asp Thr Ala Gly Asn Gly Leu Gly Glu Cys Pro Glu His
            180                 185                 190

Val Tyr Thr Val Lys Phe Thr Ala Thr Glu Leu Trp Gly Glu Gln Ser
        195                 200                 205

Gly Asp Arg His Ser Thr Val Tyr Phe Asp Val Trp Glu Pro Tyr Leu
    210                 215                 220

Ser Leu Ala Thr Ala Pro Ser Thr Arg Gly Ile
225                 230                 235

<210> 2
<211> 205
<212> PRT
<213> Rhodococcus opacus

<400> 2

Met Ala Glu Gln Arg Thr Asp Thr Gln Leu Arg Thr His Glu Glu Val
1               5                   10                  15

Val Ala Arg Val Lys Ala Leu Glu Ala Leu Leu Ile Glu Lys Gly Val
            20                  25                  30

Met Thr Thr Glu Ala Val Asp Arg Met Ala Glu Val Tyr Glu Asn Glu
        35                  40                  45

Val Gly Pro Gln Ile Gly Ala Gln Ile Val Ala Lys Ala Trp Thr Asp
    50                  55                  60

Pro Lys Phe Lys Lys Arg Leu Leu Ala Asn Ala Thr Thr Ala Cys Ala
65                  70                  75                  80

Glu Met Gly Tyr Gly Gly Leu Gln Gly Glu Asp Met Ile Val Val Glu
                85                  90                  95

Asn Thr Asp Thr Val His Asn Ala Ile Val Cys Thr Leu Cys Ser Cys
            100                 105                 110

Tyr Pro Trp Pro Val Leu Gly Leu Pro Pro Asn Trp Tyr Lys Ala Pro
        115                 120                 125

Ala Tyr Arg Ala Arg Ile Val Arg Glu Pro Arg Lys Val Leu Ala Glu
    130                 135                 140

Asp Phe Asp Phe Pro Ile Pro Asp Asp Val Glu Ile Arg Val Trp Asp
145                 150                 155                 160

Ser Ser Ala Glu Leu Arg Tyr Trp Val Leu Pro Gln Arg Pro Ala His
                165                 170                 175

Thr Glu Arg Leu Thr Glu Ser Glu Leu Val Ala Leu Val Thr Arg Asp
            180                 185                 190

Ser Met Ile Gly Val Gly Pro Val Arg Glu Ala Met Ser
        195                 200                 205